# DEMANDE DE BREVET EUROPEEN

(11) **EP 1 669 062 A1**
(43) Date de publication de la demande: **14.06.2006**
(21) Numéro de dépôt: 05292583.1
(22) Date de dépôt: 06.12.2005
(51) Int. Cl.: A61K 9/22

(54) **Comprimés permettant une liberation prolongée d'indapamide et leur procédé de préparation**

(30) Priorité: 07.12.2004 FR 0412991
(71) Demandeur: Les Laboratoires Servier, 92200 Neuilly-sur-Seine (FR)
(72) Inventeur: Debauge, Mireille, 69630 Chaponost (FR)
(74) Mandataire: Ahner, Francis

(57) **Abrégé**

La présente invention concerne un comprimé permettant la libération prolongée d'indapamide, utile notamment pour le traitement de l'hypertension artérielle essentielle, ledit comprimé ne contenant pas de polyvidone mais un mélange d'au moins deux polymères cellulosiques hydrophiles de viscosités différentes, tels que des hydroxypropylméthylcelluloses, qui permettent de contrôler cette libération prolongée, et étant éventuellement pelliculé.

## Description

La présente invention se rapporte au domaine de la formulation des médicaments.

Plus précisément, l'invention concerne la formulation de comprimés contenant de l'indapamide comme principe actif et destinés à une administration par la voie orale.

Ainsi, la présente invention vise un comprimé permettant la libération prolongée d'indapamide, utile notamment pour le traitement de l'hypertension artérielle essentielle. Ledit comprimé ne contient pas de polyvidone mais un mélange d'au moins deux polymères cellulosiques hydrophiles de viscosités différentes, tels que des hydroxypropylméthylcelluloses, qui permettent de contrôler cette libération prolongée. Ce comprimé est éventuellement pelliculé.

L'indapamide est connu pour ses propriétés diurétiques. Ce principe actif est habituellement prescrit comme agent antihypertenseur, notamment pour traiter l'hypertension artérielle essentielle.

Les documents EP 519 820 et WO 2004/002475 décrivent des formules de comprimés matriciels destinés à une administration orale et permettant la libération prolongée de l'indapamide, ainsi que leurs procédés de préparation.

Selon le brevet européen EP 519 820 (publié le 6 décembre 1995 au nom de Adir et Compagnie), la libération prolongée du principe actif est contrôlée par l'association, dans les comprimés, d'hydroxypropylméthylcellulose (HPMC) et de polyvidone, respectivement à hauteur de 30 à 50 % et de 2 à 10 % de la masse totale d'un comprimé. On observe ainsi une libération linéaire de l'indapamide pendant plus de 8 heures. En outre, 50 % de la quantité totale d'indapamide sont libérés entre 5 et 14 heures. Les taux sanguins chez l'homme sont compris entre 20 et 80 ng/ml, 24 heures au plus après administration du comprimé par voie orale.

Le brevet EP 519 820 divulgue en outre un procédé de préparation de tels comprimés matriciels, utilisant à la fois la granulation humide et la compression directe. Selon ce procédé, l'indapamide, la polyvidone et du lactose sont d'abord mélangés, puis mouillés avec une solution hydro-alcoolique de manière à obtenir une masse humide qui est ensuite granulée, séchée puis calibrée. Le granulé ainsi obtenu est mélangé à l'HPMC, puis lubrifié avec de la silice colloïdale et du stéarate de magnésium. Enfin, le mélange lubrifié est comprimé sur machine à comprimer rotative, de telle sorte que les comprimés présentent une dureté mesurée par écrasement diamétral d'environ 60 à 75 N.

La demande internationale WO 2004/002475 (publiée le 8 janvier 2004 au nom de Pliva Krakow, Zaklady Farmaceutyczne S.A.) décrit un comprimé contenant de 1,5 à 2,5 % en poids d'indapamide, de 30 à 80 % en poids de lactose monohydraté, de 2 à 10 % en poids de copovidone, de 20 à 65 % en poids d'hypromellose, et de 0,1 à 5 % en poids de lubrifiants (par exemple, du stéarate de mgnésium et/ou de la silice colloïdale anhydre). La viscosité de l'hypromellose est de 1000 à 20000 cP.

Le procédé de préparation des comprimés selon la demande WO 2004/002475 prévoit d'abord le mélange de l'indapamide avec le lactose monohydraté et la copovidone. Ensuite, le mélange est mouillé avec de l'eau purifiée, puis granulé. Le granulé ainsi obtenu est séché, refroidi, mélangé à l'hypromellose et, aux lubrifiants, puis comprimé dans une machine à comprimer.

De manière surprenante, la Demanderesse a trouvé que, contrairement à l'enseignement du brevet EP 519 820, il n'était pas nécessaire d'associer deux polymères de familles chimiques différentes, à savoir une HPMC de haute viscosité et une polyvidone, pour obtenir une libération du principe actif parfaitement contrôlée.

En effet, la Demanderesse a mis en évidence que l'association de deux polymères cellulosiques hydrophiles, tels que des HPMC, de viscosités différentes, permettait de contrôler la libération de l'indapamide d'une manière tout à fait satisfaisante.

Avantageusement, l'association de deux polymères appartenant à une même famille chimique rend la préparation des comprimés plus simple et moins coûteuse.

Ainsi, dans un premier aspect, la présente invention a pour objet un comprimé d'indapamide, notamment pour le traitement de l'hypertension artérielle essentielle, dans lequel l'indapamide est associé à des excipients permettant de contrôler sa libération prolongée. Lesdits excipients comprennent, en l'absence de polyvidone, un mélange d'au moins deux polymères cellulosiques hydrophiles de viscosités différentes, à raison de 20 à 50 % environ en poids d'un polymère de viscosité moyenne ou élevée, et de 0,5 à 10 % environ en poids d'un polymère de faible viscosité.

Dans la formule, le polymère de faible viscosité a pour fonction de lier les différents composants, tandis que le polymère de viscosité moyenne ou élevée sert à former la matrice.

La libération du principe actif se fait à la fois par diffusion et par érosion de la matrice.

Selon un mode de réalisation, le mélange d'au moins deux polymères cellulosiques hydrophiles comprend de 25 à 45 % environ, de préférence de 30 à 35 % environ, de manière encore préférée 31,51 % environ, en poids du polymère de viscosité moyenne ou élevée. ,

Dans le cadre de la présente invention, on préférera utiliser un polymère de viscosité moyenne.

Selon un autre mode de réalisation, le mélange d'au moins deux polymères cellulosiques hydrophiles comprend de 0,5 à 5 % environ, de préférence de 1 à 5 % environ, de manière encore préférée 1,66 % environ, en poids du polymère de faible viscosité.

Au sens de l'invention, les polymères cellulosiques hydrophiles sont indépendamment choisis dans le groupe comprenant les hydroxyméthylcelluloses, les hydroxyéthylcelluloses, les hydroxypropylcelluloses et les hydroxypropylméthylcelluloses.

Toutefois, il sera avantageux de n'utiliser que des hydroxypropylméthylcelluloses (HPMC).

Les HPMC sont des polymères disponibles dans le commerce, parfaitement connus de l'homme du métier et habituellement utilisés dans le domaine de la formulation des médicaments. A toutes fins utiles, on précise que de tels polymères sont notamment commercialisés sous la marque Methocel™ (distribuée par des sociétés telles que Dow Chemical Co. et Colorcon).

Une HPMC de viscosité élevée peut être choisie parmi Methocel K15M™ et Methocel K100M™, dont des solutions aqueuses à 2 % en poids présentent des viscosités respectives de 15000 et 100000 cP.

Une HPMC de viscosité moyenne peut être choisie parmi Methocel E4M™, Methocel K4M™ et Methocel K4MCR™, dont des solutions aqueuses à 2 % en poids présentent une viscosité de 4000 cP. On préférera utiliser Methocel K4MCR™.

Une HPMC de faible viscosité peut être choisie parmi Methocel E5™, Methocel E5 LV™, Methocel E15 LV™, Methocel E50 LV™, Methocel K100 LV™ et Methocel F50 LV™, dont des solutions aqueuses à 2 % en poids présentent des viscosités respectives de 5, 5, 15, 50, 100 et 50 cP. On choisira de préférence Methocel E5™ ou Methocel E5 LV™, plus préférentiellement Methocel E5 LV™, dont la viscosité d'une solution aqueuse à 2 % en poids est de 5 cP.

Selon un autre mode de réalisation, le comprimé objet de l'invention comprend, outre le principe actif et les excipients déjà décrits,
- au moins un agent de charge tel que le lactose monohydraté ; et/ou
- au moins un lubrifiant tel que le stéarate de magnésium ; et/ou
- au moins un agent de coulance tel que la silice colloïdale anhydre.

En particulier, un comprimé selon l'invention présente la formule unitaire (en mg/comprimé) suivante :

| Principe actif : | |
|---|---|
| - indapamide | 1,5 |

| Excipients : | |
|---|---|
| - lactose monohydraté | 129,00 |
| - HPMC de faible viscosité (e.g., Methocel E5 LV™) | 3,42 |
| - HPMC de viscosité moyenne (e.g., Methocel K4MCR™) | 65,00 |
| - stéarate de magnésium | 0,95 |
| - silice colloïdale anhydre | 0,41 |

Selon un autre mode de réalisation, le comprimé objet de la présente invention est pelliculé.

En particulier, le film enrobant un comprimé pelliculé selon l'invention comprend :
- au moins un agent liant tel qu'un polymère cellulosique hydrophile de faible viscosité ; et/ou
- au moins un colorant tel que le dioxyde de titane; et/ou
- au moins un plastifiant tel que le polyéthylèneglycol.

Un mode de réalisation particulier du film d'enrobage d'un comprimé pelliculé conforme à l'invention comprend (en mg/comprimé) :

| | |
|---|---|
| - HPMC de faible viscosité (e.g., Methocel E5 LV™) | 4,701 |
| - dioxyde de titane | 0,925 |
| - polyéthylèneglycol (tel que PEG 4000) | 0,383 |

Un exemple préféré d'un comprimé pelliculé selon l'invention présente la formule unitaire (en mg/comprimé) suivante :

| Principe actif : | |
|---|---|
| - indapamide | 1,5 |

| Excipients : | |
|---|---|
| - lactose monohydraté | 129,00 |
| - HPMC de faible viscosité (e.g., Methocel E5 LV™) | 3,42 |
| - HPMC de viscosité moyenne (e.g., Methocel K4MCR™) | 65,00 |
| - stéarate de magnésium | 0,95 |
| - silice colloïdale anhydre | 0,41 |

| Enrobage : | |
|---|---|
| - HPMC de faible viscosité (e.g., Methocel E5 LV™) | 4,701 |
| - dioxyde de titane | 0,925 |
| - polyéthylèneglycol (tel que PEG 4000) | 0,383 |

Un deuxième aspect de la présente invention concerne un procédé de préparation d'un comprimé d'indapamide tel que sus-décrit, qui comprend au moins les étapes suivantes :
a) mélange de l'indapamide et du polymère cellulosique hydrophile de faible viscosité ;
b) mélange du lactose et du polymère cellulosique hydrophile de viscosité moyenne ou élevée, puis mouillage avec la solution issue de l'étape a), la masse humide ainsi obtenue étant ensuite granulée, séchée puis calibrée ;
c) lubrification du granulé obtenu à l'étape b) au moyen de silice colloïdale et de stéarate de magnésium ; et
d) compression du mélange lubrifié.

De préférence, on obtient en fin de procédé des comprimés dont la dureté mesurée par écrasement diamétral va d'environ 60 à 75 N.

Selon un mode de réalisation, le procédé ci-dessus comprend en outre une étape e) d'enrobage des comprimés issus de l'étape d), de manière à former des comprimés pelliculés.

Dans ces conditions, selon un mode de réalisation, l'étape e) d'enrobage est effectuée au moyen d'un film contenant :
- au moins un agent liant tel qu'un polymère cellulosique hydrophile de faible viscosité ; et/ou
- au moins un colorant tel que le dioxyde de titane ; et/ou
- au moins un plastifiant tel que le polyéthylèneglycol.

De préférence, le film d'enrobage contient au moins un polymère cellulosique hydrophile de faible viscosité, du polyéthylèneglycol et du dioxyde de titane.

De préférence encore, le polymère de faible viscosité utilisé à l'étape e) d'enrobage est identique à celle utilisée à l'étape a) du procédé supra.

De manière particulière, le polyéthylèneglycol utilisé à l'étape e) d'enrobage est du PEG 400 ou du PEG 4000. On préférera le PEG 4000 pour ses meilleures propriétés de plastifiant.

On s'appuiera, pour illustrer la présente invention, sur les figures jointes en annexe.

Les expériences dont les résultats sont illustrés par les figures annexées ont été réalisées avec :
- un comprimé pelliculé conforme à l'exemple 1 ci-dessous (« comprimé selon l'invention ») ; et
- à titre de référence, un comprimé commercialisé en France sous la marque Fludex® 1,5 mg (comprimé pelliculé LP) par la société Euthérapie. La composition du comprimé matriciel est celle décrite dans le brevet européen EP 519 820 susmentionné (« comprimé de référence »).

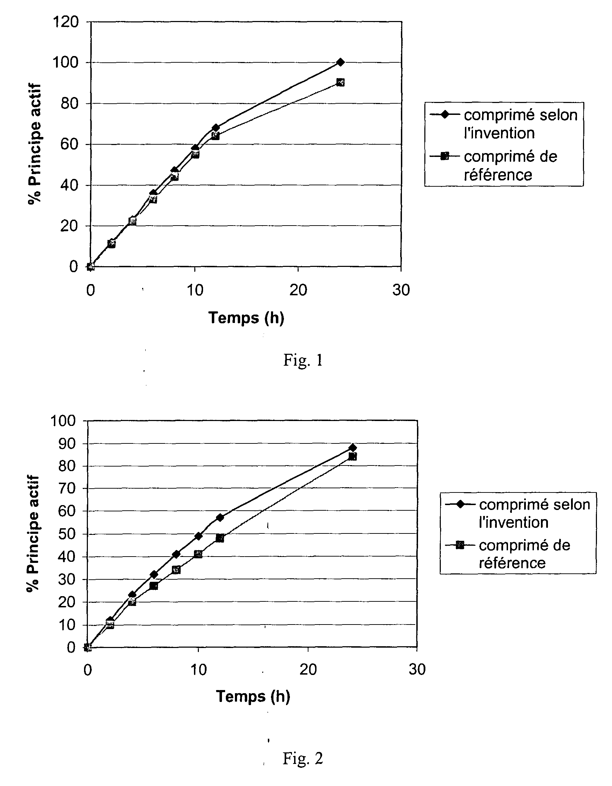

Figure 1 : cinétiques de dissolution des comprimés en milieu HCl 0,01 M.
Figure 2 : cinétiques de dissolution des comprimés en milieu HCl 0,01 M de 0 à 2 heures puis dans une solution tampon phosphate à pH 6,8.

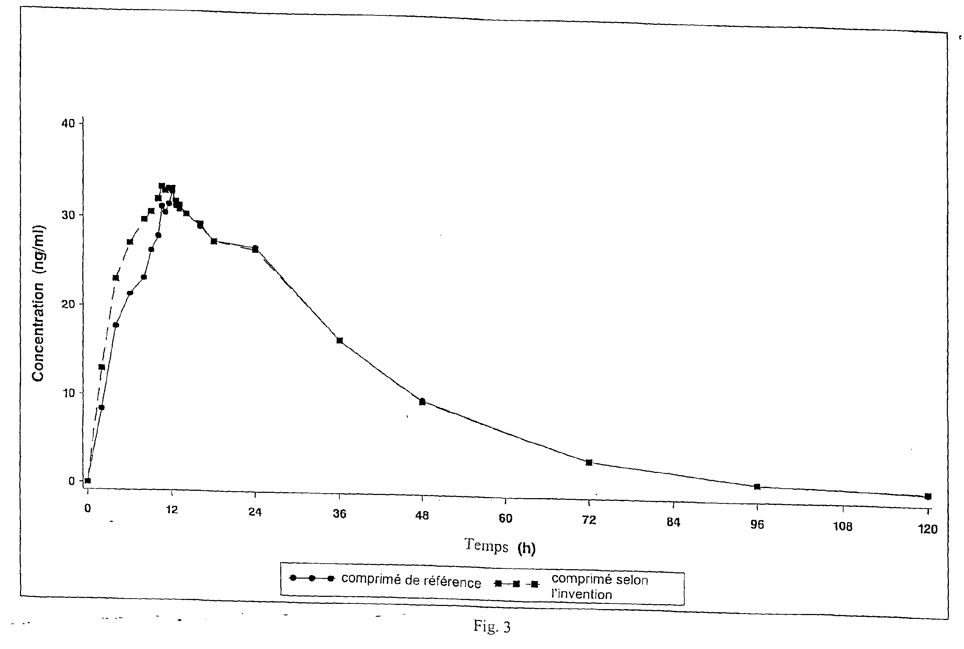

Figure 3 : concentrations sanguines d'indapamide - étude à jeûn - prise unique d'un comprimé.

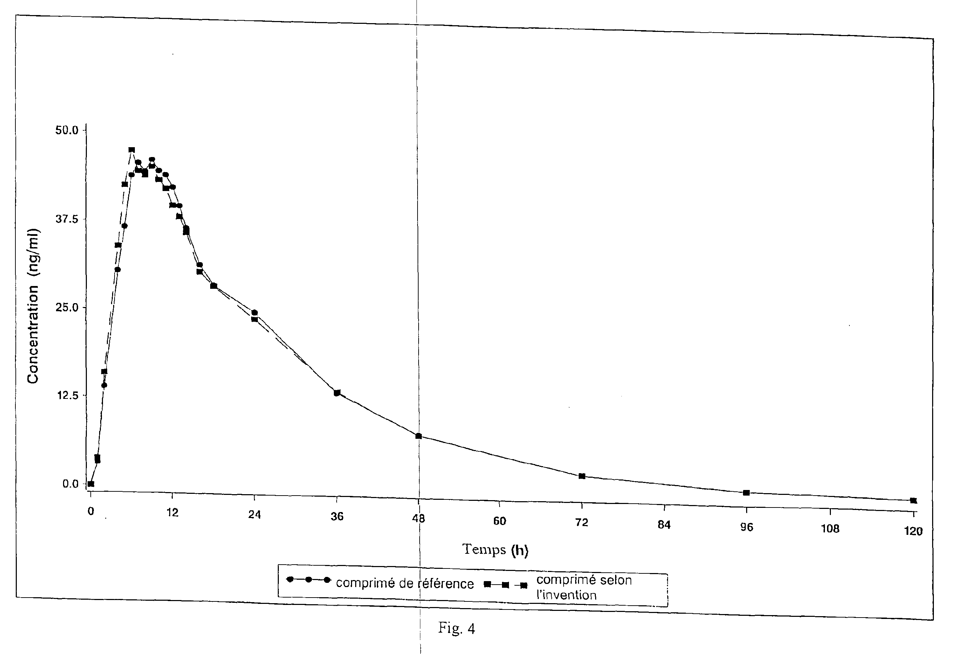

Figure 4 : concentrations sanguines d'indapamide - étude au cours d'un repas - prise unique d'un comprimé.

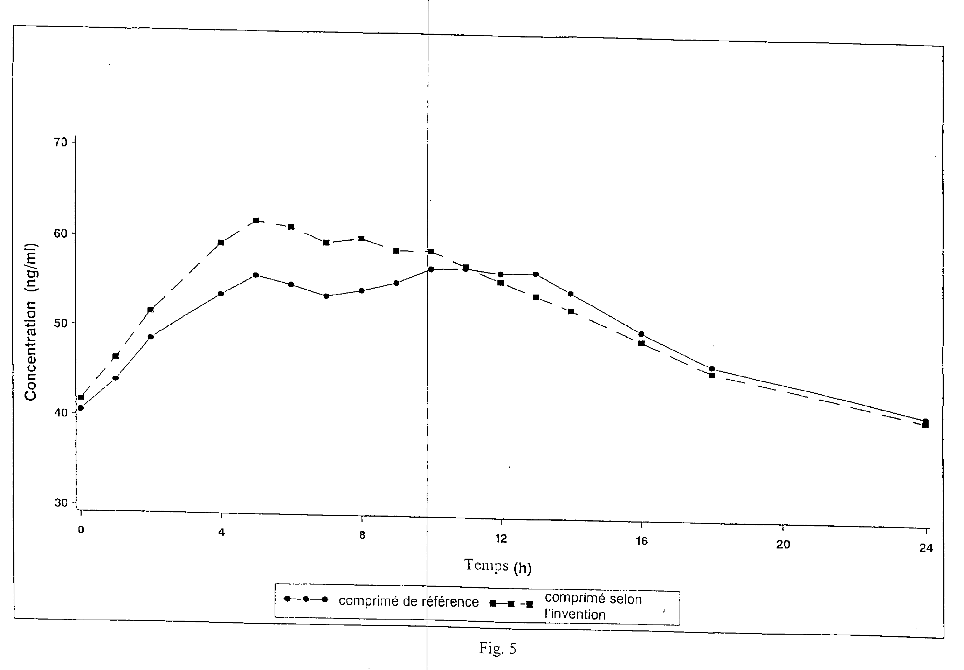

Figure 5 : concentrations sanguines d'indapamide ― dose répétée (1 comprimé par jour pendant 7 jours).

Les exemples ci-dessous concernent des modes de réalisation particuliers qui permettent d'illustrer la description générale de la présente invention. Il est bien évident que l'invention ne se limite en aucune façon auxdits exemples.

### EXEMPLES

### Exemple 1 : Exemple d'une formule unitaire d'un comprimé pelliculé selon l'invention

Le tableau 1 ci-dessous fournit la formule unitaire d'un comprimé d'indapamide 1,5 mg pelliculé (en mg/comprimé).

**Tableau 1**

| **Principe actif :** | |
|---|---|
| Indapamide | 1,5 |

| **Excipients :** | |
|---|---|
| Lactose monohydraté | 129,00 |
| HPMC (Methocel E5 LV™) | 3,42 |
| HPMC (Methocel K4MCR™) | 65,00 |
| Stéarate de magnésium | 0,95' |
| Silice colloïdale anhydre | 0,41 |

| **Enrobage :** | |
|---|---|
| HPMC (Methocel E5 LV™) | 4,701 |
| Dioxyde de titane | 0,925 |
| Macrogol (PEG 4000) | 0,383 |
| *TOTAL* | *206,29* |

### Exemple 2 : Exemple d'un procédé de préparation d'un comprimé selon l'invention

### A. Mélange de l'indapamide et du polymère cellulosique hydrophile de faible viscosité

A.1. Préparation de la solution de liaison :
   On dissout l'HPMC Methocel E5 LV™ dans de l'eau.
A.2. Préparation de la suspension d'indapamide :
   Dans un récipient en acier inoxydable pourvu d'un agitateur, on suspend l'indapamide dans une partie de la solution de liaison (environ 80%) et on mélange pendant 10 minutes.

### B. Préparation du granulé

B.1. Préparation des poudres de mélange (lactose et HPMC K4MCR™) :
   Le lactose monohydraté et l'HPMC K4MCR™ sont placés dans un granulateur et sont mélangés pendant 5 minutes.
B.2. Préparation du granulé :
   Les poudres de mélange sont mouillées dans le granulateur en présence de la solution indapamide préparée à l'étape A.2. On rince le récipient contenant la suspension d'indapamide en utilisant le reste de la solution de liaison. On passe ensuite la masse humide au tamis en utilisant des réseaux de 1000 microns. A la fin de cette opération, on transfère les granulés obtenus dans une turbine pour les sécher.
   Temps de séchage : 14 heures.
   Après séchage, on passe au tamis les granulés avec des réseaux de 1200 microns.

### C. Lubrification du granulé (mélange prêt à être comprimé)

Les granulés sont replacés dans la turbine. Du stéarate de magnésium et de la silice colloïdale anhydre sont ajoutés. L'ensemble est mélangé dans la turbine pendant 15 minutes.

### D. Préparation des comprimés

On transfère le mélange obtenu à l'étape B. dans le compartiment de charge d'une machine à comprimer rotative pour fabriquer les comprimés.
Et, facultativement :

### E. Enrobage des comprimés

E.1. Suspension d'enrobage :
   On introduit de l'eau dans un récipient en acier inoxydable pourvu d'un agitateur et d'un homogénéisateur. Sous agitation, on ajoute de l'HPMC E5 LV™ et on continue à agiter jusqu'à dissolution complète. Lorsque la dissolution est complète, on ajoute du PEG 4000 puis du dioxyde de titane, on agite et on homogénéise jusqu'à ce qu'une suspension soit obtenue.
E.2. Enrobage des comprimés :
   Le film d'enrobage est appliqué aux comprimés en rotation dans la turbine, à l'aide d'un équipement de pulvérisation automatique. A la fin de cette étape, chaque comprimé est enrobé avec environ 6 mg du film (en théorie 6,01 mg / comprimé) de manière à obtenir un poids moyen de 206,29 ± 5%.
   Apparence : Blanche, ronde, comprimé bi-convexe.
   Couleur : blanche.

## Revendications

1. Comprimé d'indapamide, dans lequel l'indapamide est associé à des excipients permettant de contrôler sa libération prolongée, **caractérisé en ce que** lesdits excipients comprennent, en l'absence de polyvidone, un mélange d'au moins deux polymères cellulosiques hydrophiles de viscosités différentes, à raison de 20 à 50 % environ en poids d'un polymère de viscosité moyenne ou élevée, et de 0,5 à 10 % environ en poids d'un polymère de faible viscosité, ledit comprimé pouvant être utilisé notamment pour le traitement de l'hypertension artérielle essentielle.

2. Comprimé selon la revendication 1, **caractérisé en ce que** ledit mélange comprend de 25 à 45 % environ, de préférence de 30 à 35 % environ, de préférence encore 31,51 % environ, en poids dudit polymère de viscosité moyenne ou élevée.

3. Comprimé selon la revendication 1 ou 2, **caractérisé en ce que** ledit polymère de viscosité moyenne ou élevée est un polymère de viscosité moyenne.

4. Comprimé selon l'une quelconque des revendications 1 à 3, **caractérisé en ce que** ledit mélange comprend de 0,5 à 5 % environ, de préférence de 1 à 5 % environ, de préférence encore 1,66 % environ, en poids dudit polymère de faible viscosité.

5. Comprimé selon l'une quelconque des revendications 1 à 4, **caractérisé en ce que** lesdits polymères cellulosiques hydrophiles sont indépendamment choisis dans le groupe comprenant les hydroxyméthylcelluloses, les hydroxyéthylcelluloses, les hydroxypropylcelluloses et les hydroxypropylméthylcelluloses.

6. Comprimé selon la revendication 5, **caractérisé en ce que** lesdits polymères cellulosiques hydrophiles sont des hydroxypropylméthylcelluloses.

7. Comprimé selon l'une quelconque des revendications 1 à 6, **caractérisé en ce qu'**il est pelliculé.

8. Comprimé selon la revendication 7, **caractérisé en ce qu'**il a pour formule unitaire (en mg/comprimé) :
| Principe actif : | |
|---|---|
| - indapamide | 1,5 |
| Excipients : | |
|---|---|
| - lactose monohydraté | 129,00 |
| - HPMC de faible viscosité (e.g., Methocel E5 LV™) | 3,42 |
| - HPMC de viscosité moyenne (e.g., Methocel K4MCR™) | 65,00 |
| - stéarate de magnésium | 0,95 |
| - silice colloïdale anhydre | 0,41 |
| Enrobage : | |
|---|---|
| - HPMC de faible, viscosité (e.g., Methocel E5 LV™) | 4,701 |
| - dioxyde de titane | 0,925 |
| - polyéthylèneglycol (tel que PEG 4000) | 0,383 |

9. Procédé de préparation d'un comprimé d'indapamide selon l'une quelconque des revendications 1 à 8, **caractérisé en ce qu'**il comprend au moins les étapes suivantes :
a) mélange de l'indapamide et du polymère cellulosique hydrophile de faible viscosité ;
b) mélange du lactose et du polymère cellulosique hydrophile de viscosité moyenne ou élevée, puis mouillage avec la solution issue de l'étape a), la masse humide ainsi obtenue étant ensuite granulée, séchée puis calibrée ;
c) lubrification du granulé obtenu à l'étape b) au moyen de silice colloïdale et de stéarate de magnésium ; et
d) compression du mélange lubrifié.

10. Procédé selon la revendication 9, **caractérisé en ce qu'**il comprend en outre une étape e) d'enrobage des comprimés issus de l'étape d).
